# EUROPEAN PATENT APPLICATION

(11) **EP 0 722 732 A1**
(43) Date of publication of application: **24.07.1996**
(21) Application number: 96300489.0
(22) Date of filing: 24.01.1996
(51) Int. Cl.: A61K 31/505, A61K 9/50

(54) **Long-acting drug formulation containing pyrimidinedione derivatives**

(30) Priority: 24.01.1995 JP 9325/95
(71) Applicant: MITSUI TOATSU CHEMICALS, INC., Tokyo (JP)
(72) Inventor: Nakajima, Takehisa, Chiba-shi, Chiba-ken (JP); Shinozaki, Hiroshi, Mobara-shi, Chiba-ken (JP); Kaneda, Manabu, Chiba-shi, Chiba-ken (JP); Hanada, Hironori, Mobara-shi, Chiba-ken (JP)
(74) Representative: Stuart, Ian Alexander

(57) **Abstract**

Treatment of arrhythmia requires administration of an antiarrhythmic drug over a long period of time. A long-acting drug formulation for oral administration, which is safe and effective for the treatment of arrhythmia, has been provided. This long-acting antiarrhythmic formulation comprises at least one unit I and at least one unit II. The unit I comprises as an effective ingredient a pyrimidinedione derivative having antiarrhythmic action and represented by the following formula (1): 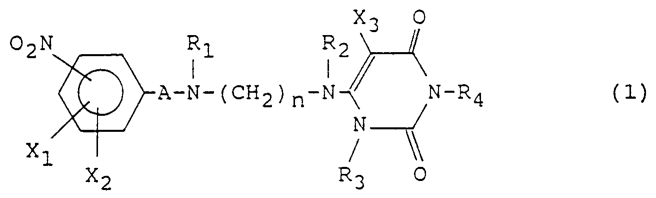
and promptly releases the effective ingredient in the stomach. On the other hand, the unit II is composed of a core, which comprises the same effective ingredient, and an enteric coat surrounding the core. The unit II promptly releases the effective ingredient in the small intestine.

## Description

This invention relates to a drug formulation, and preferably to a long-acting drug formulation, for oral administration. Still more specifically, the present invention is concerned with a pyrimidinedione drug formulation containing a pyrimidinedione derivative, preferably useful as a class III antiarrhythmic drug.

Pyrimidinedione derivatives include those useful as effective ingredients in class III antiarrhythmic drugs. For example, European Patent Publication No. 0 369 627 A2 (corresponding to U.S. Patent No. 5,008,267) discloses pyrimidinedione derivatives useful as active ingredients in drugs which show excellent effects for the treatment of arrhythmia and are also effective for the improvement of cardiac insufficiency such as heart failure.

As known techniques relating to the formation of such a pyrimidinedione derivative into medicinal preparations or drug formulations for oral administration, those disclosed in European Patent Publication No. 0 369 627 A2 (corresponding to U.S. Patent No. 5,008,267) can be mentioned. One of the Examples in this patent publication discloses a method for the formation of a drug formulation, in which granules formed by a conventional wet granulation method and containing the pyrimidinedione derivative were added with a lubricant, followed by tableting on a conventional tablet machine. The present inventors actually conducted an oral administration test with respect to this drug formulation. As a result, it was found that the blood concentration of the pyrimidinedione derivative abruptly dropped in 2 to 3 hours after administration although it was promptly absorbed and a high blood concentration was indicated in 30 minutes after administration.

As an application whose subject matter may relate to the technique according to the invention of the present application, reference may be had to European Patent Publication No. 0 640 341 Al. The techniques for the formation of drug formulations, which are disclosed in this publication, include application of a sustained release coating under an enteric coating so that the drug is not dissolved in the stomach, while the enteric coating is dissolved and the active ingredient is then gradually released through the sustained release coating in the intestine, thereby making it possible to achieve maintenance of the blood concentration at an effective level, that is, to maintain medicinal effects. Production of a drug formulation in such a form as permitting gradual release of a medicinal ingredient in the intestine has made it possible to maintain a medicinally effective blood concentration for a longer time, so that more practical drug formulations can be provided. According to a study conducted by the present inventors, it was, however, found that use of this sustained release drug formulation in an attempt to maintain the blood concentration at a still higher level within such a range as not becoming excessive involves a limit upon raising the blood concentration to such a still higher level.

Nevertheless, a long-acting drug formulation generally has many merits such as a reduction in the frequency of administration of a drug, a reduction in side effects (prevention of an excessive increase in the blood concentration) and maintenance of an effective blood concentration. Owing to the importance of these merits, a variety of long-acting drug formulations have been developed. As is described, for example, in "Saishin Yakuzaigaku (Latest Pharmaceutics), Third Edition", 331-336 (1977), Hirokawa Publishing Co., Tokyo, Japan and other publications, various long-acting drug formulations have been proposed including drug formulations in each of which a medicinal ingredient is gradually release by a chemical method or by controlled disintegration and release and those each of which composed of a gastric part and a sustained enteric release part. Some of these sustained release drug formulations have already found commercial utility.

However, these publications basically introduce nothing more than fundamental theories about long-acting drug formulations and are not considered to be such as immediately permitting provision of drug formulations useful for all pharmacological ingredients. Namely, drugs contained as active ingredients in drug formulations are different from each other in their absorption and absorbed sites in the digestive tract, their action on the behavior of the digestive tract, the extents of their first-pass effects, etc. The same long-acting method is seldom applicable equally to more than one drug formulation, so that each drug requires development of a drug formulation suited to its own properties.

### SUMMARY OF THE INVENTION

A pyrimidinedione derivative having antiarrhythmic action is a basic drug so that, when orally administered, it is promptly dissolved in the stomach and absorbed in the intestine, achieving an increase in its blood concentration in a short time after administration. However, its metabolic rate is also fast, resulting in a significant drop in its blood concentration several hours after its oral administration. If the pyrimidinedione derivative was formed into a conventional drug formulation for oral administration, prevention of an excessive increase in its blood concentration and maintenance of its blood concentration at an effective level required repeated administration of the same dose in a small quantity at intervals of several hours.

Formation of the pyrimidinedione derivative into an enteric, long-acting drug formulation as disclosed in European Patent Publication No. 0 640 341 Al for the extension of the administration interval made it possible to make still longer the tine during which the effective blood concentration would be maintained, so that its practical utility was Improved further. However, any attempt to increase the blood concentration of the medicinal ingredient to a still higher level within such a range as not becoming excessive with a view to obtaining the drug efficacy at a still higher efficiency encountered a limitation when the formulation-forming method of European Patent Publication No. 0 640 341 A1 was used.

Namely, to still more effectively use the pharmacological activity of a pyrimidinedione derivative having antiarrhythmic action, there were problems in the formation of a drug formulation specific to this compound.

A preferred embodiment of the present invention is a long-acting p.o drug formulation of a pyrimidinedione derivative having antiarrhythmic action, which is safe and effective in long-term administration required for the treatment of arrhythmia. A preferred type of embodiment may provide a novel, long-acting p.o. drug formulation comprising as an active ingredient a pyrimidinedione derivative [such as those described in European Patent publication No.0,369,627, A2 (corresponding to U.S.Patent No. 5,008,267)] which is a compound useful as a class III antiarrhythmic drug according to the classification of antiarrhythmic drugs proposed by E.M. Vaughan Williams in "Advances in Drug Research Vol. 9" edited by N.J. Harper, A.B. Simmonds, 69-101 (1974), Academic Press, London.

The present inventors proceeded with research on the absorption of pyrimidinedione derivatives having antiarrhythmic action and the maintenance of their effects. As a result, it has been found that more effective drug formulations can be provided by forming them into specific drug formulations, leading to the completion of the present invention.

A drug formulation for oral administration according to the present invention comprises at least one of each of the following unit I and unit II:
I. a gastric unit comprising as an effective ingredient a pyrimidinedione derivative having antiarrhythmic action or a pharmacologically acceptable salt thereof and permitting preferably rapid dissolution of the pyrimidinedione derivative in the stomach; and
II. an enteric unit composed of a core, which comprises as an effective ingredient a pyrimidinedione derivative having antiarrhythmic action or a pharmacologically acceptable salt thereof, and an enteric coat surrounding the core, said unit II permitting preferably rapid dissolution of the pyrimidinedione derivative in the small intestine. It is desirably long -acting.

The present invention of the above-described features has completed a technique for allowing a pyrimidinedione derivative, which has antiarrhythmic action, to promptly dissolve at a specific ratio at two sites in the digestive tract.

The present invention has therefore provided an antiarrhythmic formulation containing a pyrimidinedione derivative, which can prevent an excessive increase in the blood concentration of the derivative, can achieve stable maintenance of its blood concentration at a high level over a long time, can bring about better bioavailability for it, and can attain better maintenance of its effects.

### BRIEF DESCRIPTION OF THE DRAWINGS

FIG. 1 illustrates a dissolution profile of a capsule of Example 1;
FIG. 2 illustrates a dissolution profile of a capsule of Example 2;
FIG. 3 illustrates a dissolution profile of a capsule of Example 3;
FIG. 4 illustrates a dissolution profile of a capsule of Example 4;
FIG. 5 illustrates a dissolution profile of a capsule of Example 5;
FIG. 6 illustrates a dissolution profile of a film-coated tablet of Comparative Example 1;
FIG. 7 illustrates a dissolution profile of a capsule of Comparative Example 2;
FIG. 8 illustrates a dissolution profile of a capsule of Comparative Example 3;
FIG. 9 diagrammatically illustrates changes in dog plasma concentration following oral administration of a capsule of Example 4;
FIG. 10 diagrammatically illustrates changes in dog plasma concentration following oral administration of a film-coated tablet of Comparative Example 1;
FIG. 11 diagrammatically illustrates changes in dog plasma concentration following oral administration of a capsule of Comparative Example 2;
FIG. 12 diagrammatically illustrates changes in human plasma concentration following oral administration of capsules of Example 3;
FIG. 13 diagrammatically illustrates changes in human plasma concentration following oral administration of capsules of Example 4;
FIG. 14 diagrammatically illustrates changes in human plasma concentration following oral administration of capsules of Example 5;
FIG. 15 diagrammatically illustrates changes in human plasma concentration following oral administration of film-coated tablets of Comparative Example 1;
FIG. 16 diagrammatically illustrates changes in human plasma concentration following oral administration of capsules of Comparative Example 2; and
FIG. 17 diagrammatically illustrates changes in human plasma concentration following oral administration of capsules of Comparative Example 3.

Individual values represent average values obtained under the following conditions:

### [Dissolution tests]

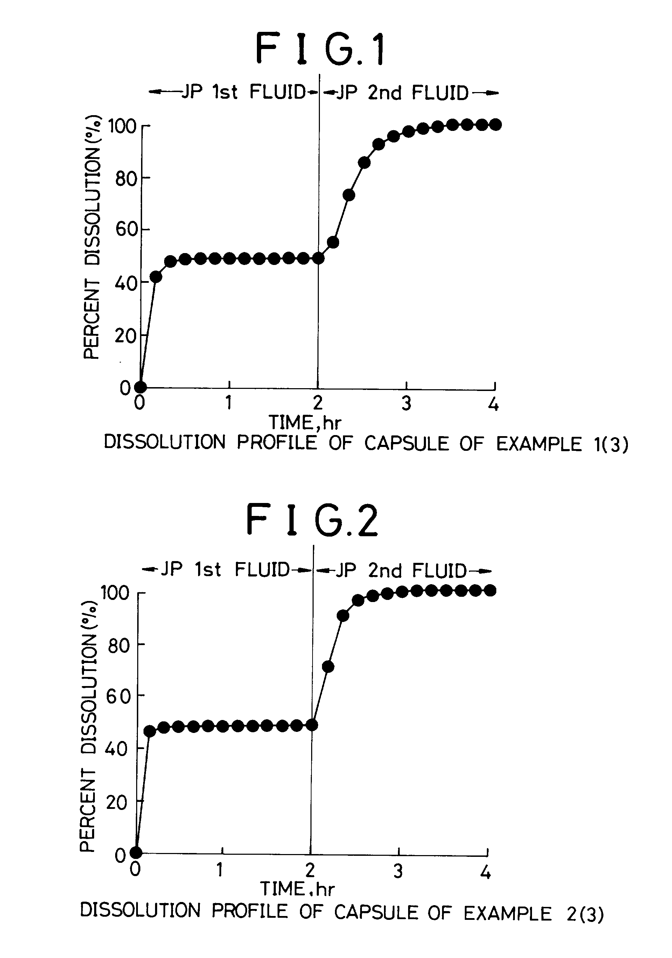

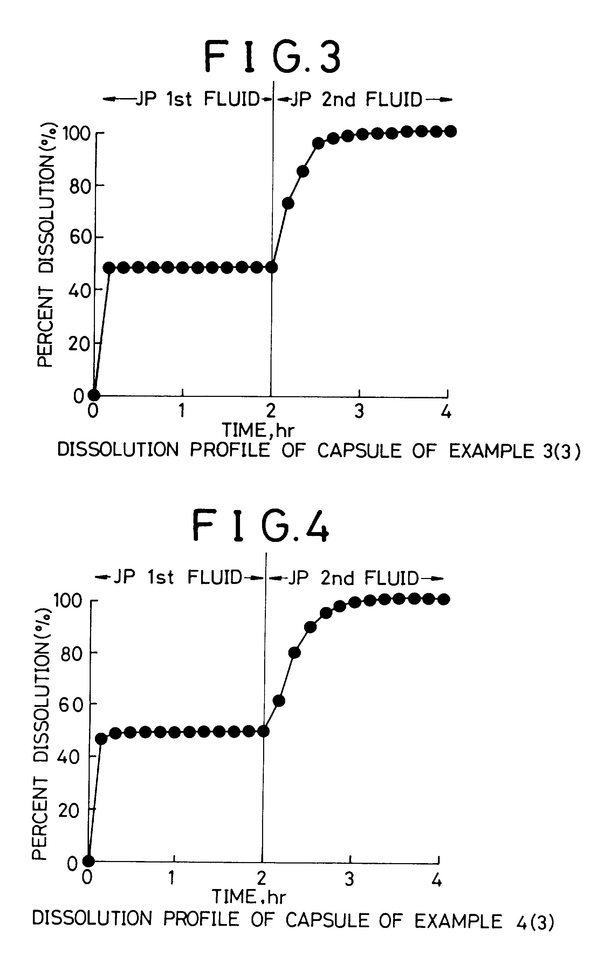

- FIG. 1 to FIG. 5:: An average value of six measurements, each conducted using one capsule.

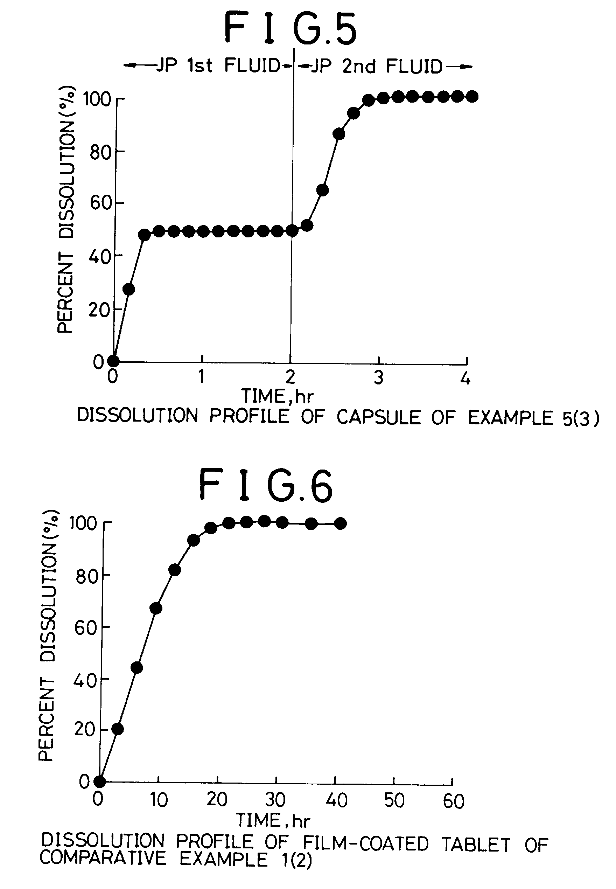

- FIG. 6:: An average value of six measurements, each conducted using one table.

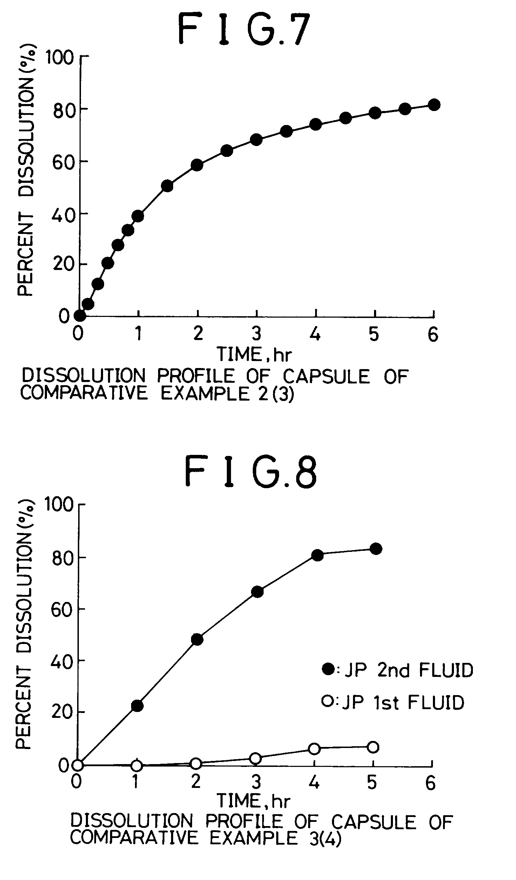

- FIG. 7:: An average value of six measurements, each conducted using one capsule.
- FIG. 8:: With respect to each of the JP 1st fluid and JP 2nd fluid, an average value of three measurements, each conducted using one capsule.

### [Changes in plasma concentration]

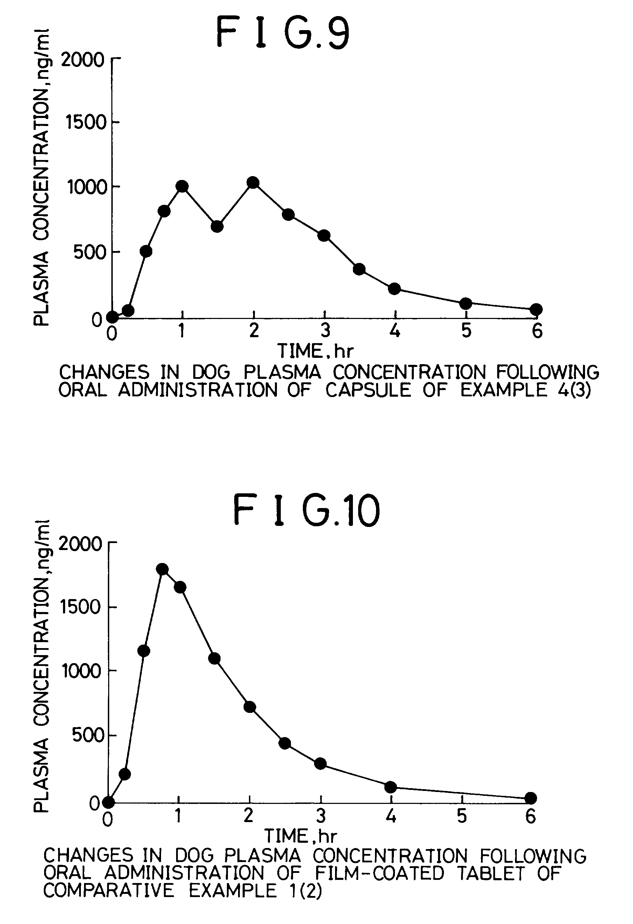

- FIG. 9 to FIG. 11:: Average values of data obtained from five dogs administered with 1 capsule or tablet per dog.

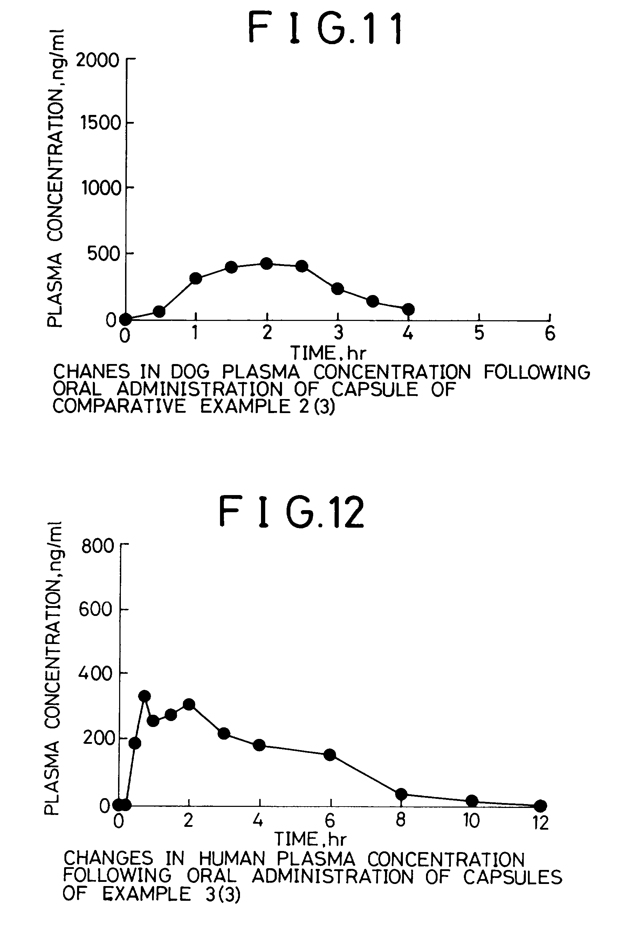

- FIG. 12:: Average values of data obtained from three human volunteers administered with 2 capsules per volunteer.

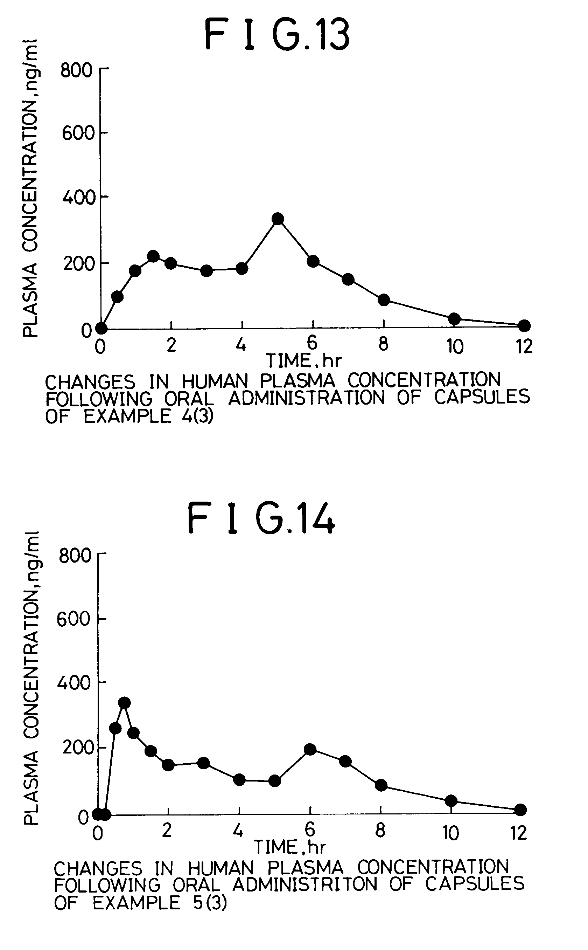

- FIG. 13 to FIG. 14:: Average values of data obtained from four human volunteers administered with 2 capsules per volunteer.

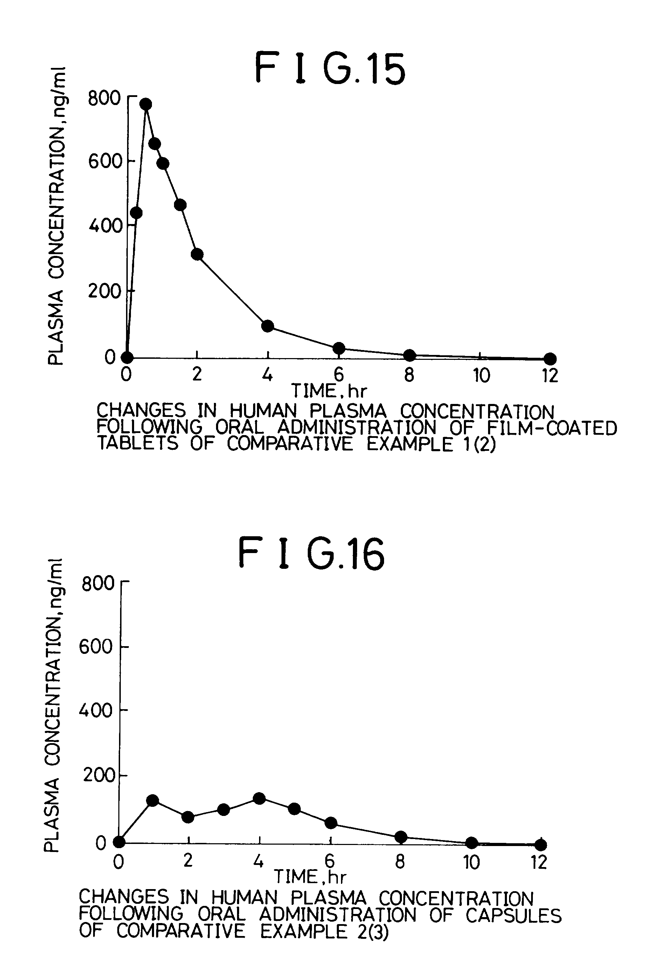

- FIG. 15:: Average values of data obtained from six human volunteers administered with 3 tablets per volunteer.
- FIG. 16:: Average values of data obtained from three human volunteers administered with 2 capsules per volunteer.

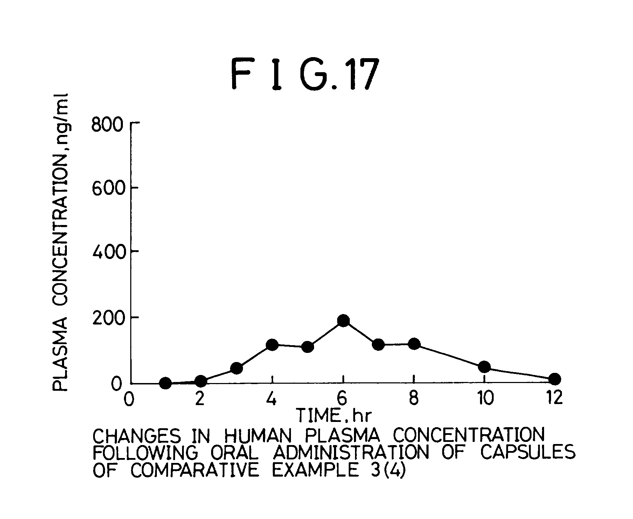

- FIG. 17: Average values of data obtained from four human volunteers administered with 3 capsules per volunteer.

### DETAILED DESCRIPTION OF THE INVENTION AND PREFERRED EMBODIMENTS

As the pyrimidinedione derivative as a pharmacologically active ingredient for the drug formulation according to the present invention, a pyrimidinedione derivative having antiarrhythmic action can be used. Illustrative examples of such a pyrimidinedione derivative include compounds represented by the following formula (1) and their pharmacologically acceptable salts. 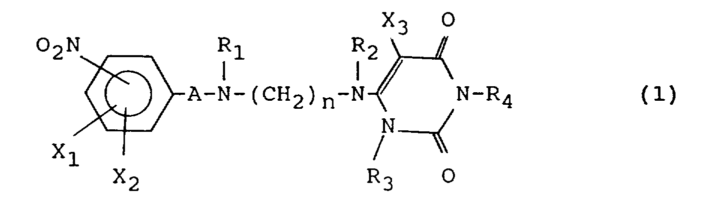 wherein A represents -(CH₂)ₘ-, -B-(CH₂)ₖ-, -D-(CH₂)_{ℓ}-, 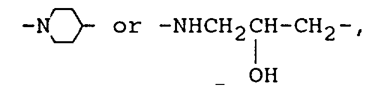 D represents 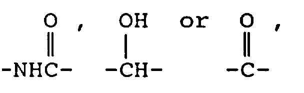 B represents an oxygen atom, a sulfur atom,

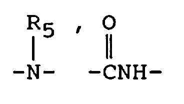

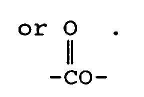

R₁ and R₂ each independently represents a hydrogen atom or a lower (for example, C₁-C₅) alkyloxycarbonyl group, an unsaturated lower (for example, C₂ or C₃) alkyl group or a lower (for example, C₁-C₅) alkyl group, one hydrogen atom of the alkyl group of said lower alkyloxycarbonyl group, unsaturated lower alkyl group or lower alkyl group being optionally substituted by a substituent group selected from the group consisting of a hydroxyl group, mono[lower (for example, C₁ or C₂) alkyl]amino groups, di[lower (for example, C₁ or C₂) alkyl]amino groups, lower (for example, C₁-C₅) alkyloxy groups, lower (for example, C₂-C₅) alkanoyloxy groups, a benzoyloxy group, halogen- or [lower (for example, C₁-C₅) alkyloxy]-substituted benzoyloxy groups, a phenyl group, halogen- or [lower (for example, C₁-C₅) alkyloxy]-substituted phenyl groups and [lower (for example, C₁-C₅) alkyloxy]carbonyl groups.

R₁ and R₂ may be coupled together into an ethylene chain or a propylene chain to form a heterocyclic ring structure.

R₃ and R₄ each independently represents a hydrogen atom or a lower (for example, C₁-C₅) alkyl group.

X₁ and X₂ each independently represents a hydrogen atom, -CO-R₆, a halogen atom, a lower (for example, C₁-C₅) alkyl group, a halogen-substituted lower (for example, C₁-C₅) alkyl group, a hydroxyl group, a lower (for example, C₁-C₅) alkyloxy group, a lower (for example, C₁-C₅) alkylthio group, a lower (for example, C₁-C₅) alkyloxycarbonyl group, a carboxyl group, a cyano group, an amino group, a lower (for example, C₂-C₅) alkanoyloxy group, a lower (for example, C₂ or C₃) alkanoylamino group, a lower (for example, C₁ or C₂) alkylsulfonamido group, a mono- or di-[lower (for example, C₁ or C₂) alkyl]amino group, a phenyl-substituted lower (for example, C₁ or C₂) alkylamino group or an unsaturated lower (for example, C₁-C₃) alkyloxy group.

X₃ represents a hydrogen atom, a nitro group, a methyl group or a cyano group.

R₅ represents a hydrogen atom, a lower (for example, C₁-C₅) alkanoyl group, a lower (for example, C₁ or C₂) alkylsulfonyl group or a lower (for example, C₁-C₅) alkyl group.

R₁ and R₅ may be coupled together into an ethylene chain or a propylene chain to form a heterocyclic ring structure.

R₆ represents a lower (for example, C₁-C₅) alkyl group, a cycloalkyl group such as a cyclopentyl or a cyclohexyl group, a phenyl group which may optionally be substituted by one to two substituent groups selected from the group consisting of halogen atoms, lower (for example, C₁-C₅) alkyl groups, a hydroxyl group and lower (for example, C₁-C₅) alkyloxy groups, or a heterocyclic group selected from pyridyl, pyrazolyl, pyrimidinyl, thienyl, furyl or pyrrolyl.

n stands for 2 or 3, m stands for an integer of 0 to 4, k stands for an integer of 2 to 4, and ℓ stands for an integer of 0 to 4.

The Ci (i: an integer of 1 to 5) exemplified above with respect to each lower alkyl group contained in the above-described substituent groups means the number of carbon atoms contained in the alkyl group.

The compounds of the formula (1) and their pharmacologically acceptable salts as well as their preparation process are disclosed, for example, in European Patent Publication No. 0 369 627 A2 (corresponding to U.S. Patent No. 5,008,267).

Illustrative of the individual units of the drug formulation according to the present invention include shaped bodies formed in such a way that they can maintain their shapes until they reach sites where they are required to dissolve or disintegrate, for example, subtilized granules, granules, powders and tablets of various shapes and sizes.

When a unit of the structure that a core containing the pharmacologically active ingredient is coated with an enteric coat is used as the unit II, it is preferred to use, as a constituent material of the enteric coat, at least one material selected from hydroxypropylmethylcellulose acetate succinate, hydroxypropylmethylcellulose phthalate, a methacrylic acid-methyl methacrylate copolymer, a methacrylic acid-ethyl acrylate copolymer, carboxymethylethylcellulose, cellulose acetate phthalate or white shellac.

The term "disintegration or disintegrate" as used herein means that a shaped body such as granules or tablets disintegrate into their constituent powder in water or a digestive fluid.

The term "dissolution or dissolve" as used herein means that a medicinal ingredient dissolves in water or a digestive fluid to form a solution.

The term "release of a medicinal ingredient" means that the medicinal ingredient, which has dissolved inside a drug formulation, flows out of the drug formulation.

The term "rapid dissolution" as used herein means that a medicinal ingredient is rapidly released as a result of rapid disintegration and dissolution of a drug formulation in water or a digestive fluid.

The term "sustained release" as used herein means that a medicinal ingredient, which has dissolved inside a drug formulation, is gradually released out of the drug formulation.

The term "long-acting" as used herein means that pharmacological action remains.

The term "release-controlled drug formulation" as used herein means a drug formulation the release of whose medicinal ingredient has been controlled by formulation-manufacturing treatment or processing such as a release-sustaining technique to avoid rapid release of the medicinal ingredient.

The term "gastric coat or coating" as used herein means a coat or coating soluble in the stomach.

The term "enteric coat or coating" as used herein means a coat or coating soluble in the intestine. Generally it is not substantially soluble in the stomach.

Illustrative examples of the form of the drug formulation according to the present invention include granules, powders, subtilized granules, tablets and capsules, each of which contains at least one each of the unit I and the unit II.

The preferably long-acting drug formulation according to the present invention comprises the gastric unit I and the enteric unit II.

It is known that in the stomach, a substance in the form of a liquid or fine particles promptly moves to the intestine, the site for its absorption, while a substance in the form of granules or tablets remain there for a certain time (2 to 3 hours). For these characteristics of the digestive tract, the gastric portion of the long-acting drug formulation according to the present invention, said gastric portion undergoing disintegration and dissolution in the stomach, is allowed to promptly move to the intestine for its absorption. On the other hand, the enteric portion which does not disintegrate and maintains its shape remains for a certain time in the stomach and is then allowed to move to the intestine for its disintegration and dissolution for absorption. It has therefore been found that single oral administration of the long-acting drug formulation according to the present invention results in rapid release of the medicinal ingredient twice at an interval of a time in the digestive tract and its absorption also takes place twice, whereby its blood concentration, that is, its effect remains.

The present inventors conducted research on changes in the blood concentration of a pyrimidinedione derivative having antiarrhythmic action by administering it to human volunteers. As a result, it was found that the bioavailability becomes higher when it is rendered rapidly dissolvable in the intestine and is caused to rapidly disintegrate and dissolve out at the site of absorption for the derivative, that is, the small intestine.

On the other hand, when combining a gastric portion with an enteric portion (or a sustained enteric release portion) both of which have heretofore been employed widely, the enteric portion (or the sustained enteric release portion) is made greater than the gastric portion, specifically, is proportioned to account about 70% in terms of the amount of a medicinal ingredient. In contrast, it has been found that in the case of the pyrimidinedione derivative, an enteric portion or a sustained enteric release portion in a proportion of 40 to 60%, that is, in a proportion substantially equal to or in some instances, smaller than an associated gastric portion shows more preferable changes in the blood concentration because the bioavailability of the derivative becomes higher when it is rendered rapidly dissolvable in the intestine.

A description will hereinafter be made of a manufacturing process of the long-acting drug formulation according to the present invention in the form of capsules.

### (1) Method for the formation of unit I:

The unit I, which is useful in the practice of the present invention, can be of any formula or composition insofar as it permits rapid dissolution of the pyrimidinedione derivative having substantial antiarrhythmic action out of the formulation. Illustrative examples of the shape of the unit I include powder, granules, pills and tablets.

The unit I can be prepared by the following methods.

The medicinal ingredient in the form of powder is used as is or as an alternative, a mixed powder is prepared, as needed, by placing a mixture of the power-form medicinal ingredient together with an excipient (for example, lactose, mannitol, crystalline cellulose, calcium hydrogenphosphate, sucrose or the like) and a disintegrator (for example, low substituted hydroxypropylcelluose, one of various starches, croscarmellose sodium, or a carboxyalkylated cellulose or an alkali metal or alkaline earth metal salt thereof) in a mixer (for example, a V-model mixer or a double cone mixer) and then rotating the mixer.

As another method, spherical granules or granules with the medicinal ingredient coated or contained therein can be formed by a method known *per se* in the art. For example, while tumbling "Nonpareil" (trademark for spherical granules of purified sucrose or spherical granules of sucrose and starch; product of Freund Industrial Co., Ltd.), "Celphere" (trademark for crystalline cellulose spheres; product of Asahi Chemical Industry Co., Ltd.) or the like as an excipient together with the medicinal ingredient in a centrifugal rolling granulator or blowing up such a granular excipient by air together with the medicinal ingredient and causing them to roll in a fluidized-bed granulator, a solution of a binder (for example, methylcellulose, hydroxypropylcellulose, hydroxypropylmethylcellulose, carmellose sodium, hydroxyethylcellulose, dextrin, pregelatinized starch, hydroxypropylstarch, pullulan, polyvinylpyrrolidone or polyvinyl alcohol) in an appropriate solvent (for example, water, ethanol, propanol, isopropanol, butanol, acetone, 2-butanone, dichloromethane, dichloroethane or a mixture thereof) is sprayed over the granules as cores, so that the medicinal ingredient or the mixture of the medicinal ingredient and the excipient and, if necessary, the disintegrator is coated.

Further, spherical granules can be formed by a conventional spray coating method. For example, while fluidizing and rolling "Nonpareil", "Celphere" or the like as an excipient in a conventional coating machine (for example, a fluidized-bed coating machine or a coating pan), a solution or suspension of the medicinal ingredient and, if necessary, an excipient, a disintegrator, a coating material (for example, talc, stearic acid or a salt thereof, or titanium dioxide), a binder and/or the like in an appropriate solvent is sprayed over granules of the excipient as cores so that the cores are coated.

As a still further method, the medicinal ingredient either as is or in the form of a composition added with an excipient and a disintegrator as needed is formed into subtilized granules or granules by a conventional dry granulation method.

As a still further method, a solution of a binder in an appropriate solvent is added to the medicinal ingredient either as is or in the form of a composition added with an excipient and a disintegrator as needed, followed by the formation into subtilized granules or granules by a conventional wet granulation method (for example, extrusion granulation, agitation granulation or fluidized-bed granulation).

Further, tablets can also be manufactured by compressing the powder, subtilized granules or granules, which have been formed by the above-described method, on a tablet machine either as are or after addition of a lubricant as needed.

Upon formation of the above-described granules or tablets, it is also possible to add, as desired, a fluidizing agent (for example, silicon dioxide hydrate, light silicic anhydride or synthetic aluminum silicate), a dispersant (for example, a hydrogenated oil, a sucrose ester of a fatty acid, or polysorbate), a gloss agent (for example, carnauba wax, cetanol or gelatin), a stabilizer (for example, an organic acid such as fumaric acid, succinic acid or DL-malic acid), and/or other adjuvants.

The spherical granules, granules or tablets, which have been obtained as described above, can be coated with gastric films as needed. They can be coated, for example, by fluidizing and rolling the spherical granules, granules or tablets in a conventional coating machine (for example, a fluidized-bed coating machine or a coating pan) and spraying a solution or suspension of a gastric coating substance [for example, hydroxypropylmethylcellulose, a methyl methacrylate-butyl methacrylate-dimethylaminoethyl methacrylate copolymer such as "Eudragit E" (polybutylmethacrylate, (2-dimethylaminoethyl)methacrylate, methylmethacrylate), or polyvinylacetal diethylaminoacetate] and, if necessary, a plasticizer (for example, macrogol, triethyl citrate or triacetine) and a coating material in an appropriate solvent over their surfaces.

In these methods, the excipient can be added in an amount of 0.01 to 5,000 parts by weight, preferably 0.05 to 2,000 parts by weight per 100 parts by weight of the medicinal ingredient in each unit. On the other hand, the disintegrator can be added in an amount of 0 to 5,000 parts by weight, preferably 0.01 to 2,000 parts by weight per 100 parts by weight of the medicinal ingredient in each unit, in which the expression "0 part by weight" means formation into the drug formulation without addition of the disintegrator. The binder can be added in an amount of 0.01 to 1,000 parts by weight, preferably 0.01 to 500 parts by weight per 100 parts by weight of the medicinal ingredient in each unit.

The lubricant can be added in an amount of 0 to 1,000 parts by weight, preferably 0.01 to 500 parts by weight per 100 parts by weight of the medicinal ingredient in each unit, in which the expression "0 part by weight" means formation into the drug formulation without addition of the lubricant.

The gastric coating material can be added in an amount of 0 to 1,000 parts by weight, preferably 0 to 500 parts by weight per 100 parts by weight of the medicinal ingredient in each unit, in which the expression "0 part by weight" means formation into the drug formulation without addition of the gastric coating material.

The preferred particle size of the unit I formed as described above ranges from 0.001 to 20 mm in diameter.

### (2) Method for the formation of unit II:

First of all, spherical granules or granules with the medicinal ingredient coated thereon or contained therein are formed by a method known *per se* in the art. Described specifically, the methods described with respect to the unit I are all applicable. For example, while rolling "Nonpareil", "Celphere" or a like excipient in a centrifugal rolling granulator or fluidizing the same in a fluidized-bed granulator, a solution of a binder in an appropriate solvent is sprayed so that the medicinal ingredient is coated on surfaces of the resultant granules. An excipient, a disintegrator and the like, which are commonly employed in the present field of art, can also be coated either together with the medicinal ingredient or individually as needed.

As another method, the medicinal ingredient can be formed into granules, if necessary, together with an excipient, a disintegrator and the like by a conventional method (for example, dry granulation or extrusion granulation).

As a further method, tablets can also be manufactured by compressing the granules, which have been formed by the above-described granulation method, on a conventional tablet machine either as are or after addition of an excipient, a lubricant and the like as needed.

The amount of the medicinal ingredient in spherical granules, granules or tablets manufactured as described above may preferably range from 0.1 to 70 parts by weight when the total amount of the unit II is assumed to be 100 parts by weight. However, the amount of the medicinal ingredient is not necessarily limited to the above range and can vary depending on the dose of the medicinal ingredient.

As a still further method, the medicinal ingredient either as is or in the form of a composition added with an excipient and a disintegrator as needed is formed into subtilized granules or granules by dry granulation.

As a still further method, a solution of a binder in an appropriate solvent is added to the medicinal ingredient either as is or in the form of a composition added with an excipient and a disintegrator as needed, followed by the formation into subtilized granules or granules by a conventional wet granulation method.

Upon formation of the above-described granules or tablets, a fluidizing agent, a dispersant, a gloss agent, a stabilizer and/or other adjuvants can also be added.

On surfaces of cores (spherical granules, granules, tablets) formed by one of the methods described above, an enteric coating material is coated. Described specifically, the unit II can be formed by spraying a suspension or solution of the enteric coating material, optionally with a plasticizer and/or a coating material, in an appropriate solvent over the surfaces of the above-described cores to coat the surfaces with the enteric coating material while fluidizing and rolling the cores in a conventional coating machine.

Examples of the enteric coating material include hydroxypropylmethylcellulose phthalate, hydroxypropylmethylcellulose acetate succinate, methacrylic acid-methyl methacrylate copolymer, methacrylic acid-ethyl acrylate copolymer, carboxymethylethylcellulose, cellulose acetate phthalate and white shellac.

The amount of the enteric coating material in the unit II formed as described above ranges from 5 to 2,000 parts by weight per 100 parts by weight of the effective ingredient in each unit. The preferred particle size of the unit II formed as described above ranges from 0.1 to 20 mm in diameter.

### (3) Formation of the long-acting drug formulation by an optimal combination of the unit I and the unit II:

Finally, the long-acting drug formulation is formed by making a set of the unit I and the unit II, which have been formed by the respective methods described above, for example, in capsules with a conventional capsule filler.

The capsule-form, long-acting drug formulation according to the present invention contains at least one, preferably 2 to 10,000 of each of the above-described unit I and unit II in a capsule. The proportion of the effective ingredient contained in the portion formed of the unit I in each capsule can range from 30 to 70%, preferably 40 to 60% of the total amount of the effective ingredient.

As capsules, conventional gelatin capsules (for example, hard capsules specified in The Pharmacopoeia of Japan, which may hereinafter be abbreviated to "JP"), starch capsules or the like can be used. The size of capsules can range from No. 00 to No. 5 having the following volumes, respectively:
No. 00 ... 0.95 mℓ No. 3 ... 0.27 mℓ
No. 0 ... 0.68 mℓ No. 4 ... 0.20 mℓ
No. 1 ... 0.47 mℓ No. 5 ... 0.13 mℓ
No. 2 ... 0.37 mℓ

One or more long-acting drug formulations according to the present invention can be administered. Further, the dose of the pyrimidinedione derivative per administration can range from 50 to 1,000 mg. Depending on the disease, the severity of its symptom, the age, the sex and the like, one to three oral administrations can be made per day.

### [Examples]

The present invention will hereinafter be described specifically by examples, comparative examples and tests. It should however be borne in mind that the present invention is by no means limited by them.

Chemical names and structural formulas of medicinal ingredients employed in the examples will be shown below, which are all embraced by the formula (1).

### Compound A: 1,3-Dimethyl-6-{4-[4-(4-nitrophenoxy)butyl]piperazin-1-yl}-2,4(1H,3H)-pyrimidinedione hydrochloride

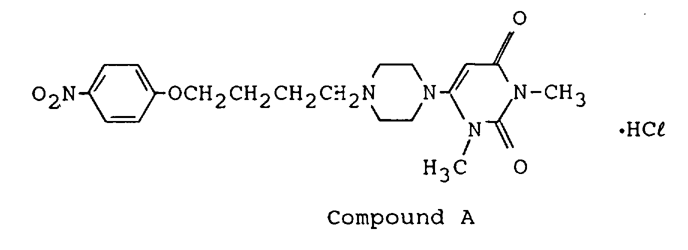

### Compound B: 1,3-Dimethyl-6-(2-[N-(2-hydroxyethyl)-3-(4-nitrophenyl)propylamino]ethylamino)-5-nitro-2,4(1H,3H)-pyrimidinedione hydrochloride

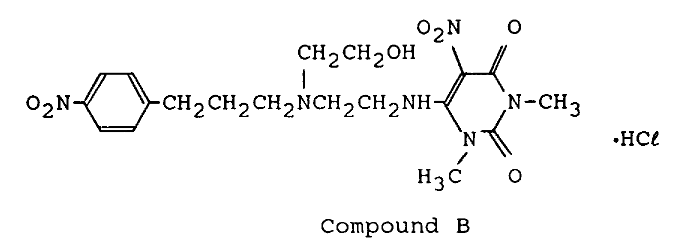

### Nifekalant hydrochloride: 1,3-Dimethyl-6-(2-[N-(2-hydroxyethyl)-N-[3-(4-nitrophenyl)propyl]amino]ethylamino)-2,4(1H,3H)-pyrimidinedione hydrochloride

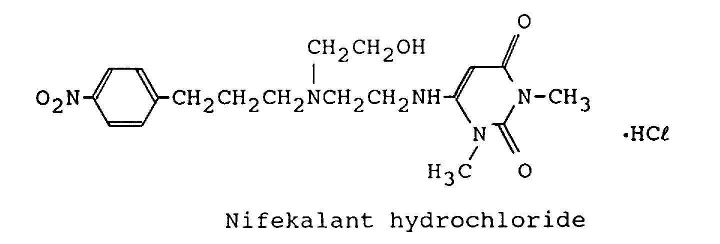

### Example 1 Granule-filled capsules (1)

### (1) Unit I:

In a centrifugal rolling granulator ("CF-360 Model", trade name; manufactured by Freund Industries Co., Ltd.; all centrifugal rolling granulators to be referred to hereinafter were the same centrifugal rolling granulator as that employed here), 240.0 g of "Nonpareil" were tumbled. A mixed powder consisting of 1,627 g of Compound A and 418 g of lactose was coated on surfaces of the tumbling "Nonpareil" granules while spraying 1,517 g of an ethanol solution (5 wt.%) of "HPC-SSL" (trade name for hydroxypropylcellulose; product of Nippon Soda Co., Ltd.). A mass of unit I was obtained in the form of granules (yield: 2,165 g).

### (2) Unit II:

Two hundred grams (200 g) of the above-obtained granules were taken and placed in a fluidized-bed coating machine ("Flow Coater Mini", trade mark; manufactured by Freund Industries Co., Ltd.; all fluidized-bed coating machines to be referred to hereinafter were the same fluidized-bed coating machine as that employed here). On the side, an enteric coating formulation was prepared by dissolving or suspending 50 g of "AQOAT" (trademark for hydroxypropylmethylcellulose acetate succinate; product of Shin-Etsu Chemical Co., Ltd.), 50 g of talc and 10 g of triethyl citrate in a mixed solvent consisting of 710 g of ethanol and 180 g of water. In the fluidized-bed coating machine, the granules were sprayed with the enteric coating formulation so that the granules were coated with enteric coats (20 parts by weight of the enteric coats per 80 parts by weight of the granules). A mass of unit II was obtained in the form of granules (yield: 250 g).

### (3) Capsules:

The mass of unit I and that of unit II were weighed in amounts of 110.1 mg and 141.5 mg, respectively, so that a portion composed of the unit I granules and a portion formed of the unit II granules individually contained 75 mg of Compound A. JP No. 2 hard capsules were each filled with them, whereby capsules were obtained.

### Example 2 Granule-filled capsules (2)

### (1) Unit I:

In the centrifugal rolling granulator, 240.0 g of "Nonpareil" were tumbled. A mixed powder consisting of 1,665 g of Compound B and 416.2 g of lactose was coated on surfaces of the tumbling "Nonpareil" granules while spraying 1,178 g of a solution of 5 parts by weight of "HPC-SSL" in a mixed solvent consisting of 5 parts by weight of water and 90 parts by weight of ethanol. A mass of unit I was obtained in the form of granules (yield: 2,140 g).

### (2) Unit II:

Two hundred grams (200 g) of the above-obtained granules were taken and placed in the fluidized-bed coating machine. On the side, an enteric coating formulation was prepared by dissolving or suspending 50 g of "AQOAT", 50 g of talc and 10 g of triethyl citrate in a mixed solvent consisting of 710 g of ethanol and 180 g of water. In the fluidized-bed coating machine, the granules were sprayed with the enteric coating formulation so that the granules were coated with enteric coats (20 parts by weight of the enteric coats per 80 parts by weight of the granules). A mass of unit II was obtained in the form of granules (yield: 248.5 g).

### (3) Capsules:

The mass of unit I and that of unit II were weighed in amounts of 109.5 mg and 139.7 mg, respectively, so that a portion composed of the unit I granules and a portion formed of the unit II granules individually contained 75 mg of Compound B. JP No. 2 hard capsules were each filled with them, whereby capsules were obtained.

### Example 3 Granule-filled capsules (3)

### (1) Unit I:

In the centrifugal rolling granulator, 700.0 g of "Nonpareil" were tumbled. Nifekalant hydrochloride (1,355 g) was coated on surfaces of the tumbling "Nonpareil" granules while spraying 1,104 g of an ethanol solution (9 wt.%) of "HPC-L" (trade name for hydroxypropylcellulose; product of Nippon Soda Co., Ltd.). A mass of unit I was obtained in the form of granules (yield: 1,949 g).

### (2) Unit II:

One hundred and fifty grams (150 g) of the above-obtained granules were taken and placed in the fluidized-bed coating machine. On the side, an enteric coating formulation was prepared by dissolving 120 g of "HPMCP" (trade mark for hydroxypropylmethylcellulose phthalate; product of Shin-Etsu Chemical Co., Ltd.) and 12 g of triethyl citrate in a mixed solvent consisting of 600 mℓ of ethanol and 1,400 mℓ of dichloromethane. In the fluidized-bed coating machine, the granules were sprayed with the enteric coating formulation so that the granules were coated with enteric coats (20 parts by weight of the enteric coats per 80 parts by weight of the granules). A mass of unit II was obtained in the form of granules (yield: 186 g).

### (3) Capsules:

The mass of unit I and that of unit II were weighed in amounts of 119.6 mg and 151.0 mg, respectively, so that a portion composed of the unit I granules and a portion formed of the unit II granules individually contained 75 mg of nifekalant hydrochloride. JP No. 2 hard capsules were each filled with them, whereby capsules were obtained.

### Example 4 Granule-filled capsules (4)

### (1) Unit I:

In the centrifugal rolling granulator, 240.0 g of "Nonpareil" were tumbled. A mixed powder consisting of 1,665 g of nifekalant hydrochloride and 416.2 g of fumaric acid was coated on surfaces of the tumbling "Nonpareil" granules while spraying 1,225 g of an ethanol solution (5 wt.%) of "HPC-SSL". A mass of unit I was obtained in the form of granules (yield: 2,263 g).

### (2) Unit II:

Two hundred grams (200 g) of the above-obtained granules were taken and placed in the fluidized-bed coating machine. On the side, an enteric coating formulation was prepared by dissolving or suspending 50 g of "AQOAT", 50 g of talc and 10 g of triethyl citrate in a mixed solvent consisting of 710 g of ethanol and 180 g of water. In the fluidized-bed coating machine, the granules were sprayed with the enteric coating formulation so that the granules were coated with enteric coats (20 parts by weight of the enteric coats per 80 parts by weight of the granules). A mass of unit II was obtained in the form of granules (yield: 250 g).

### (3) Capsules:

The mass of unit I and that of unit II were weighed in amounts of 109.4 mg and 139.6 mg, respectively, so that a portion composed of the unit I granules and a portion formed of the unit II granules individually contained 75 mg of nifekalant hydrochloride. JP No. 2 hard capsules were each filled with them, whereby capsules for the dissolution test and for administration to human were obtained.

The mass of unit I and that of unit II were weighed in amounts of 72.9 mg and 93.1 mg, respectively, so that a portion composed of the unit I granules and a portion formed of the unit II granules individually contained 50 mg of nifekalant hydrochloride. JP No. 2 hard capsules were each filled with them, whereby capsules for administration to dogs were obtained.

### Example 5 Tablet-filled capsules

### (1) Unit I:

In an agitating kneader ("Table-top New-gra Machine NG-150 Model", trade name; manufactured by Daiwa Kakoki K.K.; all agitating kneaders to be referred to hereinafter were the same agitating kneader as that employed here), 212.3 g of nifekalant hydrochloride, 61.6 g of lactose and 21.9 g of "Ac-Di-Sol" (trade name for croscarmellose sodium) were placed and, while adding dropwise 51 g of a solution of 8 parts by weight of "HPC-L" in a mixed solvent consisting of 19 parts by weight of water and 73 parts by weight of ethanol, were then agitated into granules. After the thus-obtained granules were dried at 40°C for 2 hours in a forced-air tray drier, they were sifted through a 20-mesh standard sieve to obtain granules for compression. A portion (292.2 g) of the granules was taken, to which 1.65 g of magnesium stearate were added, followed by mixing. Using a rotary tablet machine equipped with punches of 5.0 mm in diameter ("HT-P18 Model", trade name; manufactured by Hata Tekkosho K.K.; all rotary tablet machines to be referred to hereinafter were the same rotary tablet machine as that employed here), the thus-obtained mixture was compressed at a rotating speed of 30 rpm, a preliminary pressure of about 100 kg and a process pressure of about 350 kg so that tablets of unit I weighing 53.3 mg each were obtained.

### (2) Unit II:

Placed in the agitating kneader was a mixed powder which consisted of 141.5 g of nifekalant hydrochloride, 131.8 g of lactose and 121.9 g of "Ac-Di-Sol". While adding 58 g of a solution of 4.7 g of "HPC-SL" in a mixed solvent consisting of 11 g of water and 42.3 g of ethanol, the mixed powder was agitated to perform granulation. After the thus-obtained granules were dried at 40°C for 2 hours in the forced-air tray drier, they were sifted through a 20-mesh standard sieve to obtain granules for compression. A portion (291.9 g) of the granules was taken, to which 1.65 g of magnesium stearate were added, followed by mixing. Using the rotary tablet machine equipped with the punches of 5.0 mm in diameter, the thus-obtained mixture was compressed at a rotating speed of 30 rpm, a preliminary pressure of about 100 kg and a process pressure of about 350 kg so that base tablets weighing 53.3 mg each were obtained. On the side, an enteric coating formulation was prepared by dissolving or suspending 100 g of "AQOAT" and 10 g of triethyl citrate in a mixed solvent consisting of 711.1 g of ethanol and 180 g of water. In a coating pan ("Hicoater Mini", trade mark; manufactured by Freund Industries Co., Ltd.), a portion (250 g) of the base tablets was tumbled and sprayed with the enteric coating formulation. The base tablets were coated with enteric coats (9 parts by weight of the enteric coats per 91 parts by weight of the base tablets), whereby tablets of unit II were obtained.

### (3) Capsules:

JP No. 2 hard capsules were each filled with two tablets of units I and three tablets of unit II so that a portion composed of the plurality of the unit I and a portion formed of the plurality of the unit II, individually contained 75 mg of nifekalant hydrochloride, whereby capsules were obtained.

### Comparative Example 1

### Rapid-release, film-coated tablets

### (1) Base tablets:

Placed in a high-speed agitating kneader ("Vertical Granulator VG-10 Model", trade name; manufactured by Powlex) was a mixed powder which consisted of 1,538 g of nifekalant hydrochloride, 308 g of lactose and 102 g of corn starch. While adding dropwise 400 g of a solution (10 wt.%) of 40 g of "HPC-L" in a mixed solvent consisting of 72 g of water and 288 g of ethanol, the mixed powder was agitated to perform granulation. After the thus-obtained granules were dried at 40°C for 4 hours in the forced-air tray drier, they were sifted through a 16-mesh standard sieve to obtain granules for compression. A portion (1,969 g) of the granules was taken, to which 12.3 g of magnesium stearate were added, followed by mixing. Using the rotary tablet machine equipped with punches of 7.0 mm in diameter, the thus-obtained mixture was compressed at a rotating speed of 40 rpm, a preliminary pressure of about 180 kg and a process pressure of about 600 kg so that base tablets weighing 130.0 mg each were obtained.

### (2) Rapid-release film-coated tablets:

A portion (1,900 g) of the thus-obtained base tablets was placed in a coating pan ("Hicoater FC-48 Model", trade name; manufactured by Freund Industries Co., Ltd.). On the side, a gastric coating formulation was prepared by dissolving or suspending 129 g of hydroxypropylmethylcellulose, 39 g of titanium oxide and 12 g of macrogol in a mixed solvent consisting of 810 g of ethanol and 50 g of water. The base tablets were sprayed with the gastric coating formulation, so that the base tablets were coated with gastric films (4 parts by weight of the gastric films per 96 parts by weight of the base tablets). Rapid-release film-coated tablets were hence obtained.

### Comparative Example 2 Sustained-release granules

### (1) Base granules:

In the centrifugal rolling granulator, 1,500 g of "Nonpareil" were tumbled. Nifekalant hydrochloride (1,500 g) was coated on surfaces of the tumbling "Nonpareil" granules while spraying 630 g of an ethanol solution (9.2 wt.%) of "HPC-L". Base granules were hence obtained (yield: 2,813 g).

### (2) Sustained-release granules:

In the fluidized-bed coating machine, a portion (200 g) of the above-obtained base granules was placed and air was fed to fluidize the base granules. The base granules were sprayed with a sustained-release coating formulation which had been prepared by dissolving 22.5 g of ethylcellulose and 2.5 g of triacetine in a mixed solvent consisting of 350 mℓ of ethanol and 150 mℓ of water. The base granules were hence coated with sustained-release films (4 parts by weight of the sustained-release films per 96 parts by weight of the base granules), whereby sustained-release granules were obtained (yield: 207 g).

### (3) Sustained-release capsules:

JP No. 2 hard capsules were each filled with 321.3 mg of the above-obtained sustained-release granules containing 150 mg of nifekalant hydrochloride, whereby sustained-release capsules for the dissolution test and for administration to human were obtained.

JP No. 2 hard capsules were each filled with 214.1 mg of the above-obtained sustained-release granules containing 100 mg of nifekalant hydrochloride, whereby sustained-release capsules for administration to dogs were obtained.

### Comparative Example 3

### Sustained enteric release granules

### (1) Base granules:

Nifekalant hydrochloride (2,000 g) and fumaric acid (1,000 g) were mixed for 15 minutes in a V-model mixer ("V-10 Model", trade name; manufactured by Tokuju Kosakusho Co., Ltd.). In a fluidized-bed granulator ("FL0-5", trade name; manufactured by Freund Industries Co., Ltd.), the resultant mixture was granulated while spraying 2,670 g of an aqueous solution (15 wt.%) of polyvinyl alcohol. After the thus-formed granules were dried at 45°C for 30 minutes in the fluidized-bed granulator, the granules were ground into powder of about 20 micrometers in average particle size in a hammer mill ("KII-1", trade name; manufactured by Fuji Paudal Co., Ltd.). In the centrifugal rolling granulator, 500 g of "Nonpareil" were then tumbled and, while spraying a mixed solvent consisting of 50 parts by weight of water and 50 parts by weight of ethanol, the above-obtained powder were sprinkled over the tumbling "Nonpareil" granules as cores, whereby base granules of about 1.3 mm in average particle size were obtained (yield: 1,016 g).

### (2) Sustained-release granules:

In an agitating fluidized-bed granulating coater ("New-marmerizer-Labo Model", trade name; manufactured by Fuji Paudal Co., Ltd.), a portion (120 g) of the above-obtained base granules was placed and air was fed to fluidize the base granules. The base granules were sprayed with a sustained-release coating formulation which had been prepared by dissolving or suspending 7.4 g of "Eudragit RS" (trade name for polyethylacrylate, methylmethacrylate, trimethylammonioethylmethacrylatechlorid; product of Röhm Pharma), 0.4 g of "Plasdone K-30" (trade name for polyvinylpyrrolidone; product of ISP Japan Ltd.), 0.4 g of magnesium stearate and 0.8 g of triethyl citrate in a mixed solvent consisting of 109.6 g of ethanol and 17.4 g of water. The base granules were hence coated with sustained-release films (7 parts by weight of the sustained-release films per 93 parts by weight of the base granules), whereby sustained-release granules were obtained (yield: 127.5 g).

### (3) Sustained enteric release granules:

In the agitating fluidized-bed granulating coater, 120 g of the above-obtained sustained-release granules were placed and air was fed to fluidize the base granules. The sustained-release granules were then sprayed with an enteric coating formulation which had been prepared by dissolving 18.3 g of HPMCP and 2.0 g of triethyl citrate in a mixed solvent consisting of 77.3 g of ethanol and 309.4 g of dichloromethane. The base granules were hence coated with enteric coats (14.5 parts by weight of the enteric coats per 85.5 parts by weight of the sustained-release granules), whereby sustained enteric release granules were obtained (yield: 140.0 g).

### (4) Sustained enteric release capsules:

JP No. 2 hard capsules were each filled with 252.5 mg of the above-obtained sustained enteric release granules, whereby sustained enteric release capsules were obtained.

### [Tests]

Representative test results will hereinafter be described to show advantageous effects of the present invention.

Incidentally, the term "absorption test" means a test in which subsequent to administration of an effective ingredient to an organism, the plasma concentration of the effective ingredient is measured at predetermined time intervals and Cmax, Tmax and AUC are determined from the resulting time versus plasma concentration curve. The Cmax, Tmax and AUC in the absorption test have the following meanings:
- Cmax:: Concentration peak of a plasma concentration curve.
- Tmax:: Time until the concentration peak of the plasma concentration curve was reached.
- AUC:: Area under the plasma concentration curve.

### Dissolution tests:

Dissolution testing method 1: With respect to each of the capsules of Example 1(3), Example 2(3), Example 3(3), Example 4(3) and Example 5(3), a dissolution test using one capsule was conducted following the Dissolution Test I (the rotating basket method) specified in the Pharmacopoeia of Japan, Eleventh Edition. Described specifically, after tested at 37°C and 100 rpm for 2 hours in 900 mℓ of the JP 1st fluid, the dissolving fluid was immediately changed to 900 mℓ of the JP 2nd fluid, followed by further testing at 37°C and 100 rpm.

Test results: The results of the tests are shown in FIG.1 to FIG. 5.

Dissolution testing method 2: With respect to each of the film-coated tablets of Comparative Example 1(2) and the capsules of Comparative Example 2(3), a dissolution test using one capsule or one tablet was conducted following the Dissolution Test II (the paddle method) specified in the JP. The test was conducted at 37°C and 100 rpm in 900 mℓ of the JP 1st fluid.

Test results: The results of the tests are shown in FIG. 6 to FIG. 7.

Dissolution testing method 3: With respect to the capsules of Comparative Example 3(4), a dissolution test using one capsule was conducted following the Dissolution Test II (the paddle test) specified in the JP. Using the JP 1st fluid and the JP 2nd fluid (each 900 mℓ), respectively, the test was conducted at 37°C and 100 rpm.

Test results: The results of the test are shown in FIG. 8.

### Absorption tests:

Absorption testing method 1: With respect to each of the capsules of Example 4(3), the film-coated tablets of Comparative Example 1(2) and the capsules of Comparative Example 2(3), five beagles (weight: 9 to 12 kg) which had been fasted overnight were orally administered with the capsules or film-coated tablets to give a dose of 100 mg in terms of nifekalant hydrochloride. After the administration of one capsule or one tablet, blood samples were periodically collected from a forelimb vein. Subsequent to centrifugation, the concentration of the effective ingredient in the plasma was measured by high performance liquid chromatography.

Test results: Average values of changes in the plasma concentration are shown in FIG. 9 to FIG. 11, while average values of the pharmacokinetic parameters are presented in Table 1 and Table 2. Incidentally, the "1st" and "2nd" under each of Cmax and Tmax in Table 1 indicate values of plasma concentration peaks emanated from a gastric rapid-release portion and an enteric rapid-release portion, which correspond to two peaks observed on the corresponding plasma concentration change curve, respectively.

**Table 1**

| Pharmacokinetic Parameters of Dog Plasma Concentration (Example) | | | | | | | |
|---|---|---|---|---|---|---|---|
| Drug formulation | Dose* (mg) | | Cmax(ng/mℓ) | | Tmax(hr) | | AUC |
| | Gastric portion | Enteric portion | 1st | 2nd | 1st | 2nd | (ng·hr/mℓ) |
| Ex. 4(3) | 50 | 50 | 1025 | 1493 | 1.0 | 2.0 | 2806 |

| | | | | | | | |
|---|---|---|---|---|---|---|---|
| *: Nifekalant hydrochloride | | | | | | | |

**Table 2**

| Pharmacokinetic Parameters of Dog Plasma Concentration (Comparative Examples) | | | | |
|---|---|---|---|---|
| Drug formulation | Dose* (mg) | Cmax (ng/mℓ) | Tmax (hr) | AUC (ng·hr/mℓ) |
| Comp. Ex. 1(2) | 100 | 1856 | 0.9 | 3040 |
| Comp. Ex. 2(3) | 100 | 542 | 1.9 | 1076 |

| | | | | |
|---|---|---|---|---|
| *: Nifekalant hydrochloride | | | | |

Absorption testing method 2: With respect to each of the various drug formulations of Example 3(3), Example 4(3), Example 5(3), Comparative Example 1(2), Comparative Example 2(3) and Comparative Example 3(4), three to six normal volunteers (male, age: 20 to 25 years old, weight: 55 to 65 kg) were orally administered with the drug formulation to give a dose of 300 mg in terms of nifekalant hydrochloride using the following doses:
Example 3(3) ... 2 capsules
Example 4(3) ... 2 capsules
Example 5(3) ... 2 capsules
Comparative Example 1(2) ... 3 tablets
Comparative Example 2(3) ... 2 capsules
Comparative Example 3(4) ... 3 capsules
After the administration, blood samples were periodically collected from a forelimb vein. Subsequent to centrifugation, the concentration of the effective ingredient in the plasma was measured by high performance liquid chromatography.

Test results: Average values of changes in the plasma concentration are shown in FIG. 12 to FIG. 17, while average values of the pharmacokinetic parameters are presented in Table 3 and Table 4. Incidentally, the "1st" and "2nd" under each of Cmax and Tmax in Table 3 indicate values of plasma concentration peaks emanated from a gastric rapid-release portion and an enteric rapid-release portion, which correspond to two peaks observed on the corresponding plasma concentration change curve, respectively.

**Table 3**

| Pharmacokinetic Parameters of Human Plasma Concentration (Examples) | | | | | | | |
|---|---|---|---|---|---|---|---|
| Drug formulation | Dose* (mg) | | Cmax(ng/mℓ) | | Tmax(hr) | | AUC |
| | Gastric portion | Enteric portion | 1st | 2nd | 1st | 2nd | (ng·hr/mℓ) |
| Ex. 3(3) | 150 | 150 | 341 | 266 | 0.8 | 3.3 | 1500 |
| Ex. 4(3) | 150 | 150 | 235 | 370 | 1.4 | 5.5 | 1706 |
| Ex. 5(3) | 150 | 150 | 377 | 344 | 0.7 | 5.5 | 1790 |

| | | | | | | | |
|---|---|---|---|---|---|---|---|
| *: Nifekalant hydrochloride | | | | | | | |

**Table 4**

| Pharmacokinetic Parameters of Human Plasma Concentration (Comparative Examples) | | | | |
|---|---|---|---|---|
| drug formulation | Dose* (mg) | Cmax (ng/mℓ) | Tmax (hr) | AUC (ng·hr/mℓ) |
| Comp. Ex. 1(2) | 300 | 942 | 0.5 | 1622 |
| Comp. Ex. 2(3) | 300 | 108 | 3.0 | 754 |
| Comp. Ex. 3(4) | 300 | 306 | 6.0 | 986 |

| | | | | |
|---|---|---|---|---|
| *: Nifekalant hydrochloride | | | | |

As a result of the dissolution tests conducted in accordance with the dissolution testing method 1, namely, from FIG. 1 to FIG. 5, it was found that in the case of the JP 1st fluid, the percent dissolution of the drug formulation of each of the Examples remained substantially unchanged after about 50% of the total amount of the medicinal ingredient had dissolved out 20 minutes after the initiation of the test. It was also found that the medicinal ingredient dissolved out up to about 80% or more of its total amount 30 minutes after the change to the JP 2nd fluid and dissolved out almost entirely 1 hour after the change to the JP 2nd fluid. It has therefore been confirmed that the drug formulation of each of the Examples has both rapid gastric dissolvability and rapid enteric dissolvability.

As a result of the dissolution tests conducted in accordance with the dissolution testing method 2, namely, from FIG. 6 and FIG. 7, it has been confirmed that the film-coated tablets of Comparative Example 1(2) were a rapid-release drug formulation whereas the capsules of Comparative Example 2(3) were a sustained-release drug formulation.

As a result of the dissolution test conducted in accordance with the dissolution testing method 3, namely, from FIG. 8, the capsules of Comparative Example 3(4) were found to remain substantially free from disintegration and dissolution in the JP 1st fluid and to show sustained-release property in the JP 2nd fluid. Accordingly, they have been confirmed to be a sustained enteric release drug formulation.

The results of the absorption tests conducted in accordance with the absorption testing method 1 are shown in FIG. 9 to FIG. 11 and also in Table 1 and Table 2. When the film-coated tablets of Comparative Example 1(2) were administered to the beagles, the plasma concentration reached the Cmax in about 1 hour after the administration but after that, the medicinal ingredient promptly vanished from the plasma. In contrast, when the capsules of Example 4(3) according to the present invention were administered to the beagles, two plasma peak concentrations appeared 1 hour and 2 hours after the administration, respectively, regarding the pharmacohinetic parameters in Tables 1 and 2. These two concentration peaks were found to be controlled low compared with the Cmax of the film-coated tablets of Comparative Example 1(2). Further, compared with the plasma concentration of the film-coated tablets of Comparative Example 1(2), the plasma concentration of the capsules of Example 4(3) remained higher from about 2 hours after the administration, thereby indicating maintenance of a higher plasma concentration. Regarding AUC, no difference was observed between the film-coated tablets of Comparative Example 1(2) and the capsules of Example 4(3). Upon administration of the capsules of Comparative Example 2(3) to the beagles, on the other hand, substantial reductions in both Cmax and AUC were observed compared with the capsules of Example 4(3).

From the above-described results of the absorption tests conducted using the beagles in accordance with the absorption testing method 1, administration of a drug formulation according to the present invention is expected to suppress an excessive increase in Cmax and also to maintain a high blood concentration. The drug formulation according to the present invention is therefore expected to be usable as a safe and effective drug formulation.

With a view to confirming, through human volunteers, the results of the absorption tests which had been conducted using the beagles in accordance with the absorption testing method 1, tests were conducted in accordance with the absorption testing method 2. The results are shown in FIG. 12 to FIG. 17 and also in Table 3 and Table 4. Upon administration of the film-coated tablets of Comparative Example 1(2) to the human volunteers, the plasma concentration reached the Cmax in 0.5 hour after the administration but after that, the medicinal ingredient promptly vanished from the plasma. In contrast, when the drug formulation of each of the Examples according to the present invention were administered to the human volunteers, two plasma concentration peaks appeared 0.7 to 1.4 hours and 3.3 to 5.5 hours after the administration, respectively, regarding the pharmacokinetic parameters in Tables 3 and 4. These two concentration peaks were found to be controlled low compared with the Cmax of the film-coated tablets of Comparative Example 1(2). It was also found that compared with the plasma concentration of the film-coated tablets of Comparative Example 1(2), the plasma concentration of the drug formulation of each of the Examples remained higher from about 4 hours after the administration, thereby indicating maintenance of a higher plasma concentration. With respect to the capsules of Example 4(3), it has been confirmed that the blood concentration remains longer compared with the results of the absorption tests conducted using the beagles in accordance with the absorption testing method 1. Regarding AUC, no difference was observed between the film-coated tablets of Comparative Example 1(2) and the drug formulations of the Examples, thereby confirming similarity in bioavailability. Upon administration of the capsules of Comparative Example 2(3) to the human volunteers, on the other hand, substantial reductions in both Cmax and AUC were observed compared with the drug formulations of the Examples. Further, upon administration of the capsules of Comparative Example 3(4) to the human volunteers, the Cmax indicated substantially the same value as those obtained by the administration of the drug formulations of the Examples, but the plasma concentration generally remained low and AUC was also found to drop to 1/2 to 2/3 or so of those achieved by the drug formulations of the Examples. From the results of the absorption tests of the capsules of Comparative Example 2(3) and the capsules of Comparative Example 3(4), it has been suggested that in the case of a drug formulation which gradually releases its medicinal ingredient in the stomach or in the intestine, administration of the effective ingredient in a greater dose makes it possible to achieve a similar AUC and also to maintain a similar blood concentration as the drug formulations of the Examples. This approach is however by no means desired from the standpoint of an expense to be incurred on patients, to say nothing of the safety.

From the foregoing, the long-acting drug formulation according to the present invention has been confirmed that it can avoid an excessive increase in blood concentration, can maintain a high blood concentration, and is also excellent in bioavailability. The long-acting drug formulation according to the present invention can be furnished as a long-acting safe p.o. drug formulation effective for the treatment of arrhythmia, which requires long-term administration.

## Claims

1. A drug formulation for oral administration, comprising at least one of each of the following unit I and unit II.
I. a unit comprising as an effective ingredient a pyrimidinedione derivative or its pharmacologically acceptable salt having antiarrhythmic action and permitting dissolution of the pyrimidinedione derivative in the stomach; and
II. a unit composed of a core, which comprises as an effective ingredient a pyrimidinedione derivative or its pharmacologically acceptable salt having antiarrhythmic action, and an enteric coat surrounding the core, said unit II permitting dissolution of the effective ingredient in the small intestine.

2. The drug formulation according to claim 1, wherein the enteric coat of the unit II comprises at least one material selected from hydroxypropylmethylcellulose acetate succinate, hydroxypropylmethylcellulose phthalate, a methacrylic acid-methyl methacrylate copolymer, a methacrylic acid-ethyl acrylate copolymer, carboxymethylethylcellulose, cellulose acetate phthalate or white shellac.

3. The drug formulation according to claim 1 or 2 wherein the pyrimidinedione derivative is represented by the following formula (1): 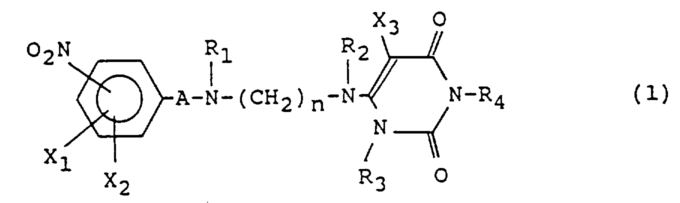 wherein A represents -(CH₂)ₘ-, -B-(CH₂)ₖ-, -D-(CH₂)_{ℓ}-*,* 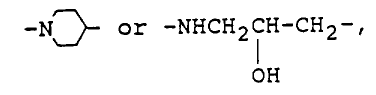 D represents 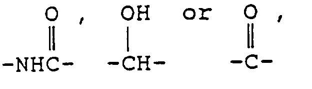 B represents an oxygen atom, a sulfur atom, 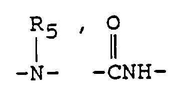 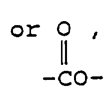 R₁ and R₂ each independently represents a hydrogen atom or a lower alkyloxycarbonyl group, an unsaturated lower alkyl group or a lower alkyl group, one hydrogen atom of the alkyl group of said lower alkyloxycarbonyl group, unsaturated lower alkyl group or lower alkyl group being optionally substituted by a substituent group selected from the group consisting of a hydroxyl group, mono(lower alkyl)amino groups, di(lower alkyl)amino groups, lower alkyloxy groups, lower alkanoyloxy groups, a benzoyloxy group, halogen- or (lower alkyloxy)-substituted benzoyloxy groups, a phenyl group, halogen- or (lower alkyloxy)-substituted phenyl groups and (lower alkyloxy)carbonyl groups, R₁ and R₂ may be coupled together into an ethylene chain or a propylene chain to form a heterocyclic ring structure, R₃ and R₄ each independently represents a hydrogen atom or a lower alkyl group, X₁ and X₂ each independently represents a hydrogen atom, -CO-R₆, a halogen atom, a lower alkyl group, a halogen-substituted lower alkyl group, a hydroxyl group, a lower alkyloxy group, a lower alkylthio group, a lower alkyloxycarbonyl group, a carboxyl group, a cyano group, an amino group, a lower alkanoyloxy group, a lower alkanoylamino group, a lower alkylsulfonamido group, a mono- or di-(lower alkyl)amino group, a phenyl-substituted lower alkylamino group or an unsaturated lower alkyloxy group, X₃ represents a hydrogen atom, a nitro group, a methyl group or a cyano group, R₅ represents a hydrogen atom, a lower alkanoyl group, a lower alkylsulfonyl group or a lower alkyl group, R₁ and R₅ may be coupled together into an ethylene chain or a propylene chain to form a heterocyclic ring structure, R₆ represents a lower alkyl group, a cycloalkyl group, a phenyl group which may optionally be substituted by one to two substituent groups selected from the group consisting of halogen atoms, lower alkyl groups, a hydroxyl group and lower alkyloxy groups, or a heterocyclic group selected from pyridyl, pyrazolyl, pyrimidinyl, thienyl, furyl or pyrrolyl, n stands for 2 or 3, m stands for an integer of 0 to 4, k stands for an integer of 2 to 4, and ℓ stands for an integer of 0 to 4.
